# FASCICULE DE BREVET EUROPEEN

(11) **EP 2 355 740 B1**
(45) Date de publication et mention de la délivrance du brevet: **18.05.2016**
(21) Numéro de dépôt: 09799666.4
(22) Date de dépôt: 09.12.2009
(51) Int. Cl.: A61B 18/20

(54) **APPAREIL A MAIN DE SOINS CORPORELS A DETECTION DE PRESENCE**
TRAGBARE KÖRPERPFLEGEVORRICHTUNG MIT ANWESENHEITSERKENNUNG
HANDHELD BODY CARE APPLIANCE WITH PRESENCE DETECTION

(30) Priorité: 11.12.2008 FR 0858479
(43) Date de publication de la demande: 17.08.2011
(73) Titulaire: SEB S.A., 69130 Ecully (FR)
(72) Inventeur: GAILHARD, Thierry, F-69970 Chaponnay (FR); LEGRAIN, Marc, F-01390 Civrieux en Dombes (FR); CHAMBON, Vincent, F-69510 Soucieu en Jarrest (FR)
(74) Mandataire: Guéry-Jacques, Géraldine
(86) Numéro de dépôt international: PCT/FR2009/001405
(87) Numéro de publication internationale: WO 2010/066966

(56) Documents cités:
- EP-A- 1 923 014
- WO-A1-96/23447
- JP-A- 6 205 732
- US-A1- 2007 185 553

## Description

La présente invention concerne le domaine technique des appareils à main de soins corporels tel que notamment les épilateurs mécaniques, les appareils de traitement des poils par rayon laser, les appareils de dépose de couche de cire d'épilation ou encore les appareils de massage.

De tels appareils de soins corporels comprennent généralement une tête de traitement équipée de moyens de traitement activés électriquement et destinés à être placés au contact ou à proximité de la peau dans la zone à traiter. Dans certains cas, il est souhaitable notamment pour des raisons de sécurité de s'assurer de la présence de la peau à proximité des moyens de traitement avant d'en déclencher le fonctionnement. Tel est notamment le cas lorsque les moyens de traitement mettent en oeuvre une source laser dont le rayonnement peut être dangereux pour la vue. Afin de répondre à cette problématique, une demande internationale WO 02078559 a proposé de mettre en oeuvre des moyens de détection de la présence de la peau au niveau de la tête de traitement pour n'activer le fonctionnement de la source laser qu'en cas de proximité effective de la peau. La mise en oeuvre d'un tel système de détection de proximité permet de garantir que le rayonnement laser ne puisse pas être émis sans qu'un obstacle, en l'occurrence la peau, ne l'intercepte. Toutefois, cette seule détection de proximité de la peau ne permet pas de garantir le fonctionnement des moyens de traitement seulement dans le cadre d'une utilisation volontaire et la simple détection de la proximité de la peau n'interdit pas une mise en marche de l'appareil alors qu'il est seulement en appui sur une partie du corps de la personne en cours de soin, l'utilisateur n'en surveillant pas le fonctionnement. La demande US2007/0185553 A1 divulgue un appareil à main de soins corporels comprenant un corps qui comprend une zone de compréhension. Cet appareil comprend aussi une tête de traitement qui équipe le corps et qui est destinée à être appliquée contre ou à proximité de la peau et qui est équipée de moyens de traitement activés électriquement. Cet appareil comprend également des moyens de détection de proximité de la peau avec la tête de traitement. Une unité de commande est adaptée pour autoriser le fonctionnement des moyens de traitement si la peau est détectée au niveau de la tête de fonctionnement et si conjointement un interrupteur est pressé. L'interrupteur peut être utilisé pour sélectionner les modes d'utilisation par des successives. Donc l'interrupteur ne peut pas détecter la présence de la main au niveau de la zone de préhension. En effet l'utilisateur peut avoir pressé enterrupteur et posé l'appareil, avant de l'utiliser.

Il apparaît donc le besoin d'un appareil de soins corporels qui ne présente pas cet inconvénient et qui puisse garantir que le fonctionnement des moyens de traitement intervient uniquement lors d'une utilisation effective et volontaire de l'appareil.

Afin d'atteindre cet objectif, un objet de l'invention est un appareil à main de soins corporels comprenant :
- un corps qui comprend une zone de préhension,
- une tête de traitement qui équipe le corps et qui est destinée à être appliquée contre ou à proximité de la peau et comprend des moyens de traitements activés électriquement,
- des moyens de détection de la proximité d'une peau avec la tête de traitement,
- une unité électronique de commande qui est adaptée pour au moins autoriser le fonctionnement des moyens de traitement si de la peau est détectée à proximité de la tête de traitement,

Selon l'invention, l'appareil comprend des moyens de détection de la présence d'une main sur la zone de préhension et l'unité électronique de commande est adaptée pour au moins autoriser le fonctionnement des moyens de traitement si de la peau est détectée à proximité de la tête de traitement et si conjointement une main est détectée au niveau de la zone de préhension.

Par la mise en oeuvre de ces conditions cumulatives pour autoriser le fonctionnement des moyens de traitement, l'invention réduit plus encore les risques de démarrages intempestifs accidentels et augmente la sécurité de l'appareil par rapport à la sécurité mettant en oeuvre la seule détection de la proximité de la peau au niveau de la tête de traitement. De plus, la double détection de la main de l'utilisateur au niveau de la zone de préhension et de la peau à proximité de la tête de traitement peut être utilisée pour déclencher le fonctionnement des moyens de traitement sans que l'utilisateur ait à actionner un interrupteur mécanique. Ainsi, selon une caractéristique de l'invention, l'unité électronique de commande est adaptée pour déclencher le fonctionnement des moyens de traitement si de la peau est détectée à proximité de la tête de traitement et si conjointement une main est détectée au niveau de la zone de préhension. Cette caractéristique de l'invention offre alors un très grand confort d'utilisation à l'appareil selon l'invention.

De plus, il est à noter que la détection de la proximité de la peau permet, lorsque l'utilisateur a en main l'appareil mais l'éloigne de la zone à traiter, un arrêt des moyens de traitement permettant ainsi d'économiser de l'énergie électrique, ce qui est avantageux lorsque l'appareil est alimenté par des batteries. Il en résulte une augmentation substantielle de l'autonomie de l'appareil notamment lorsque le traitement réalisé implique des passes successives ou des déplacements le long d'une zone à traiter uniquement dans un sens de sorte que l'utilisateur est amener à éloigner la tête de traitement de la zone à traiter de façon intermittente tout au cours de son processus de soins corporels. Tel est notamment le cas lors de l'utilisation d'un épilateur mécanique dont le sens de passage dépend du sens de pousse des poils. L'arrêt résultant de l'éloignement de la zone à traiter permet également d'offrir un meilleur confort sonore à l'utilisateur lorsque les moyens de traitement comprennent des pièces mécaniques en mouvement.

L'appareil sera autorisé à fonctionner tant que la peau est détectée à proximité de la tête de traitement et conjointement tant qu'une main est détectée au niveau de la zone de préhension. Ces deux conditions sont cumulatives pour autoriser le fonctionnement de l'appareil.

Selon une autre caractéristique de l'invention, l'unité électronique de commande est adaptée pour autoriser ou déclencher le fonctionnement des moyens de traitement si de plus la présence de la main est détectée avant la présence ou la proximité de la peau. Un tel mode de fonctionnement de l'appareil permet d'augmenter encore la sûreté de son fonctionnement dans la mesure où des moyens de traitement ne pourront être mis en oeuvre qu'après une prise en mains de l'appareil puis placement de la tête de traitement sur ou à proximité de la zone à traiter. Ainsi, dans l'éventualité où l'appareil à main de soins corporels selon l'invention serait posé sur une surface assimilée par les moyens de détection de proximité à de la peau pour ensuite être pris en main par l'utilisateur, l'unité de commande détecterait que la détection de proximité de peau est intervenue avant la détection d'une main sur la zone de préhension, de sorte que des moyens de traitement ne seraient pas activés.

Selon l'invention, la détection de proximité peut être réalisée de toute façon appropriée et, par exemple, par la mise en oeuvre d'un système de palpeur électromécanique. Selon une forme de réalisation, les moyens de détection de proximité comprennent au moins une source de lumière émettant en direction d'une zone de détection de proximité située au niveau de la tête de traitement et au moins un capteur de la lumière en provenance de la zone de détection de proximité. La mise en oeuvre de tels moyens de détection de proximité sans contact permet une grande fiabilité des moyens de détection dans la mesure où ils ne mettent en oeuvre aucune pièce mécanique mobile.

La mise en oeuvre de tels moyens optiques de détection de proximité permet également une simplification de leur implantation sur l'appareil. Ainsi, selon une variante de réalisation, la source de lumière et le capteur de lumière, des moyens de détection de proximité, sont situés à l'intérieur du corps à distance de la tête de traitement. L'appareil comprend alors au moins un guide de lumière interposé entre, d'une part, la source de lumière et le capteur et, d'autre part, la zone de détection. Ainsi, il est possible d'implanter la source de lumière et son capteur associé sur une carte électronique de l'appareil qui portera également l'unité électronique de commande. Cette forme d'implantation permet donc de simplifier le processus de fabrication de l'appareil selon l'invention. Le guide de lumière peut être réalisé de toute manière appropriée telle que par exemple sous la forme d'un faisceau de fibre optique. Le guide de lumière peut également être constitué par une ou plusieurs pièces en matière plastique aux caractéristiques optiques adaptées. Selon une caractéristique de l'invention, la tête de traitement est amovible et le guide de lumière comprend alors une première partie solidaire du corps et une deuxième partie solidaire de la tête de traitement. La mise en oeuvre d'un guide de lumière en deux parties permet d'obtenir un système supportant un grand nombre d'opérations de montage et de démontage en offrant une fiabilité supérieure à celle qui serait offerte par un système de raccordement électrique.

Selon l'invention, les moyens de détection de présence de la main peuvent également être réalisés de toute manière appropriée telle que par exemple sous la forme de détecteurs électromécaniques. Selon une forme de réalisation, les moyens de détection de présence comprennent au moins une source de lumière émettant en direction d'une zone de détection de présence située au niveau de la zone de préhension et au moins un capteur de la lumière en provenance de la zone de détection de présence.

Selon une variante de réalisation, la source de lumière et le capteur de lumière, des moyens de détection de présence, sont situés à l'intérieur du corps à distance de la zone de préhension et l'appareil comprend alors au moins un guide de lumière interposé entre, d'une part, la source de lumière et le capteur et, d'autre part, la zone de préhension. Cette variante de réalisation permet de simplifier la fabrication de l'appareil selon l'invention en autorisant une implantation de la source de lumière et du capteur associé sur la carte de l'appareil.

Selon une variante de réalisation, les moyens de détection de proximité et les moyens de détection de présence possèdent une source de lumière en commun. L'utilisation d'une source de lumière commune aux moyens de détection de proximité et aux moyens de détection de présence permet de réduire les coûts de production du dispositif selon l'invention.

Selon une variante de réalisation, les moyens de détection de proximité et les moyens de détection de présence comprennent des moyens pour mesurer la résistance utilisant un matériau conducteur du type polymère conducteur, permettant d'établir une connexion électrique avec l'unité de contrôle. En particulier, l'utilisation d'une résistance commune aux moyens de détection de proximité et aux moyens de détection de présence permet de réduire les coûts de production du dispositif selon l'invention.

Selon une variante de réalisation, les moyens de détection de proximité et les moyens de détection de présence possèdent des moyens pour mesurer la capacitance incluant une antenne ou un autre capteur capacitif.

Selon une variante de réalisation, les moyens de détection de proximité et les moyens de détection de présence possèdent des moyens pour mesurer la température comprenant des transducteurs thermiques.

Selon l'invention, l'appareil de soins corporels peut être adapté pour assurer différents types de soins, notamment des soins à caractères esthétiques ou de bien-être. Ainsi, l'appareil de soins corporels selon l'invention peut être adapté pour réaliser des massages ou de la stimulation et correspondre à des appareils du type de ceux décrits par la demande FR 2 746 320.

L'appareil de soins corporels selon l'invention peut également être adapté pour assurer l'application de cire à épiler et former un appareil tel que décrit par la demande FR 2 733 672.

L'appareil selon l'invention peut aussi être adapté pour réaliser une épilation mécanique et ainsi constituer un appareil du type de ceux décrits par les demandes FR 2 823 080, FR 2 810 215 et FR 2 804 844. Dans ce dernier cas, les moyens de traitement sont alors adaptés pour assurer une telle épilation mécanique.

L'appareil selon l'invention peut aussi être adapté pour réaliser une épilation par exposition à des radiations magnétiques, par exemple une épilation laser et ainsi constituer un appareil du type de ceux décrits dans la demande WO9623447.

Bien entendu, les différentes caractéristiques, formes et variantes de réalisation de l'invention peuvent être associées les unes avec les autres selon diverses combinaisons dans la mesure où elles ne sont pas incompatibles ou exclusives les unes des autres.

Par ailleurs, diverses autres caractéristiques et avantages de l'invention ressortent de la description annexée effectuée en référence aux dessins qui illustrent des formes non limitatives de réalisation d'un appareil à main de soins corporels selon l'invention.
La figure 1 est une coupe schématique d'un appareil de soins corporels selon l'invention conçu pour l'épilation mécanique.
La figure 2 est une coupe schématique d'un appareil de soins corporels conforme à l'invention conçu pour assurer un massage corporel selon une technique dite du palper-rouler.

Sur ces figures, les références communes désignent des éléments communs aux différentes formes de réalisation.

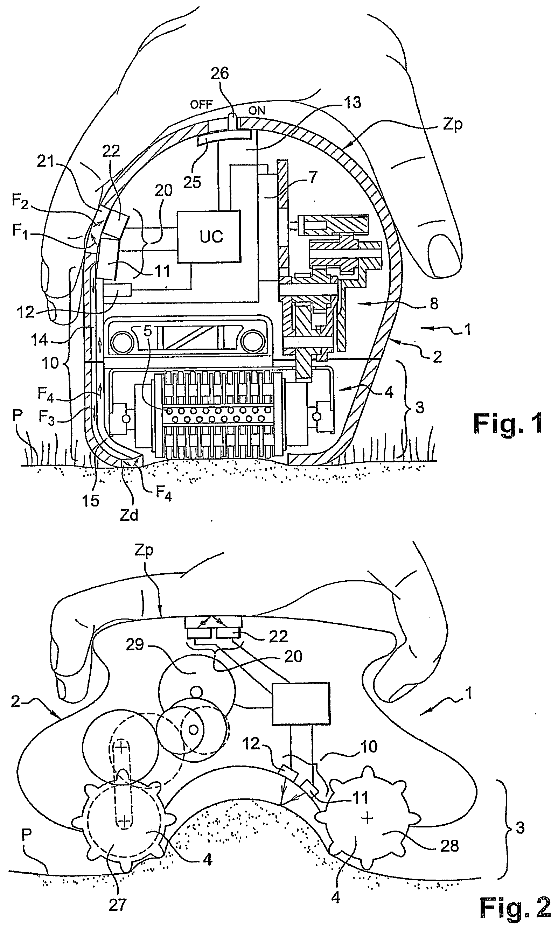

Un appareil de soins corporels selon l'invention, tel qu'illustré à la figure 1 est désigné dans son ensemble par la référence 1, comprend un corps 2 pourvu d'une zone de préhension Zp adaptée pour permettre une prise en main de l'appareil 1 par un utilisateur. L'appareil 1 comprend également une tête de traitement 3 qui équipe le corps 2. La tête de traitement 3 peut faire partie intégrante du corps 2 ou au contraire, comme selon l'exemple illustré, être adaptée sur ce dernier en étant amovible. La tête de traitement 3 comprend des moyens de traitement 4 activés électriquement. La tête de traitement 3 est alors adaptée pour être appliquée contre ou à proximité de la peau P d'un utilisateur ou d'une personne dont il est pris soin de manière que les moyens 4 puissent y effectuer le traitement pour lequel ils ont été conçus.

Selon l'exemple illustré, les moyens de traitement 4 sont adaptés pour réaliser une épilation mécanique et comprennent à cet effet un rouleau 5 portant une pluralité de pinces d'épilation réparties à sa périphérie et sur sa longueur et s'ouvrant et se fermant au fur et à mesure de la rotation du rouleau 5. Ce rouleau 5 est alors entraîné en rotation par un moteur électrique 7 via un train d'engrenages 8. Le mode de réalisation du rouleau 5 et de son entraînement est bien connu de l'homme du métier, pour être notamment décrit par la demande FR 2 804 844, et ne nécessite donc pas une plus ample description.

Le moteur électrique 7 est piloté par une unité d'électronique de commande UC qui est alimentée en énergie électrique soit par un cordon, soit par des batteries non représentées. Selon l'invention, l'appareil de soins corporels 1 comprend en outre des moyens 10 de détection de la peau P à proximité ou au contact de la tête de traitement 3. Selon l'exemple illustré, les moyens de détection de proximité 10 sont conçus pour assurer une détection optique. Les moyens 10 comprennent alors une source de lumière 11 pilotée par l'unité électronique de commande. La source de lumière 11 peut être de toute nature appropriée et par exemple, comprendre au moins une diode électroluminescente (DEL ou LED en anglais) émettant un rayonnement infrarouge. Les moyens de détection de proximité 10 comprennent également un capteur de lumière 12 raccordé à l'unité de commande UC et adapté pour être sensible au rayonnement émis par la source de lumière 11. Selon l'exemple illustré, la source de lumière 11 et le capteur 12 sont implantés sur une même carte électronique 13 qui supporte en outre l'unité de commande UC. La carte est située à l'intérieur du corps 2 à distance de la tête de traitement 3 et plus particulièrement de la zone de contact de cette dernière avec la peau P. Les moyens de détection de proximité 10 comprennent alors au moins une et, selon l'exemple illustré, deux guides de lumière dont un premier 14 est adapté pour acheminer la lumière en provenance de la source 11 au niveau d'une zone de détection Zd tandis que le deuxième 15 est adapté pour acheminer la lumière en provenance de la zone de détection Zd à destination du capteur 12. Dans la mesure où la tête de traitement 3 est, selon l'exemple illustré, amovible, les guides de lumière 14 et 15 comprennent chacun deux parties dont l'une est solidaire de la tête de traitement 3 et l'autre du corps 2. Les deux parties de chaque guide de lumière 14 ou 15 sont alors adaptées pour assurer une continuité de transmission de la lumière lorsque la tête de traitement 3 est adaptée sur le corps 2.

L'appareil 1 comprend également des moyens de détection de la présence d'une main au niveau de la zone de préhension Zp. Selon l'exemple illustré, les moyens de détection de présence 20 comprennent une source de lumière qui dans le cas présent est la même diode électroluminescente 11 que la diode électroluminescente constitutive de la source de lumière des moyens de détection de proximité 10. Cette source commune de lumière 11 est alors située d'une part en regard de l'entrée du guide de lumière 14 et d'autre part en regard d'une fenêtre de détection 21 aménagée dans le corps 2 et ouverte au niveau de la zone de préhension Zp. Les moyens de détection de présence 20 comprennent en outre un capteur de lumière 22 sensible à la lumière émise par la source 11 et donc, selon l'exemple illustré, sensible au rayonnement infrarouge. Le capteur 22 est alors raccordé à l'unité électronique de commande UC. La fenêtre peut, selon l'invention être vide ou au contraire formée par un guide de lumière 21 assurant un couplage optique entre une partie de la surface extérieure de la zone de préhension d'une part et la source 11 et le capteur 22 d'autre part.

L'appareil de soins corporels 1 comprend en outre, selon l'exemple illustré un interrupteur mécanique 25 comprenant un curseur 26 mobile entre une position « off » et une position « on ». L'interrupteur 25 permet alors une coupure générale de l'alimentation de l'appareil 1.

L'appareil de soins corporels 1 selon l'invention ainsi constitué fonctionne de la manière suivante.

Lorsqu'un utilisateur souhaite procéder à une épilation d'une zone de peau, il place le curseur 26 de l'interrupteur 25 en position « on ». Ensuite, l'utilisateur prend en main le corps 2 de l'appareil en laissant dégagée la tête de traitement 3 comme cela est illustré à la figure 1. Une partie au moins de la main de l'utilisateur, dans le cas présent l'index de ce dernier, se trouve en regard de la fenêtre 21 de sorte que le rayonnement issu de la source 11 traverse la fenêtre 21 pour se réfléchir sur la peau et être dirigé vers le capteur 22 comme le montre les flèches F₁ et F₂. L'unité de commande UC qui pilote le fonctionnement de la source 11 et se trouve reliée au capteur 22 est alors en mesure de conclure à la présence de la main de l'utilisateur. Il est à noter qu'en l'absence d'obstacles au niveau de la fenêtre 21 et notamment d'un doigt, le rayonnement lumineux en provenance de la source 11 ne serait pas réfléchi de sorte que le capteur 22 ne détecterait aucun rayonnement et l'unité de commande UC conclurait à l'absence de main sur la zone de préhension.

Après cette prise en main, l'utilisateur applique la tête de traitement 3 contre la peau P au niveau de la zone à traiter. Le rayonnement issu de la source 11 est acheminé par le guide de lumière 14 au niveau de la zone de détection Zd et se trouve alors réfléchi sur la peau P en direction du guide de lumière 15 qui achemine ce rayonnement réfléchi jusqu'au capteur 12, comme le montrent les flèches F₃ et F₄. L'unité de commande UC en déduit alors la présence de la peau et, dans la mesure où la présence de la main est également détectée, autorise le fonctionnement du moteur électrique 7. De la même manière que pour les moyens de détection de présence, les moyens de détection de proximité sont conçus de manière qu'en l'absence de peau au niveau de la zone de détection Zd, les rayons lumineux en provenance de la source 11 ne sont pas reflétés de sorte que le capteur 12 ne reçoit aucun flux lumineux réfléchi en provenance de la source 11. Cette absence de flux lumineux au niveau du capteur 12 est interprétée par l'unité de commande UC comme une absence de peau.

Selon l'exemple illustré, l'unité de commande UC déclenche ainsi le fonctionnement du moteur de manière à entraîner le rouleau 5.

Lorsque l'utilisateur décolle la tête de traitement 3 de la peau P, soit pour arrêter la séquence d'épilation, soit pour effectuer une nouvelle passe, l'unité de commande UC pilote l'arrêt du fonctionnement du moteur 7. Cet arrêt intervient soit immédiatement afin de réaliser une économie d'énergie et de réduire les bruits aériens liés à la rotation du moteur soit après une temporisation d'une durée prédéfinie de manière que la tête de traitement soit toujours en mouvement si l'utilisateur réapplique l'appareil de traitement sur la peau juste après l'avoir relevé de manière à assurer une nouvelle passe. Cette temporisation empêche des marches arrêts répétés en cours d'épilation prolongée et évite ainsi la lassitude de l'utilisateur.

Selon l'invention, l'unité de commande peut autoriser le fonctionnement des moyens de traitement indépendamment de l'ordre de la détection de la préhension et de la proximité de la zone à traiter ou au contraire en tenant compte de cet ordre de détection. Dans ce dernier cas, l'unité de commande UC autorisera le fonctionnement des moyens de traitement uniquement si la détection de la préhension intervient avant la détection de la proximité de la peau à traiter.

Selon l'invention, l'appareil de soins corporels n'est pas nécessairement un appareil d'épilation. Ainsi, la figure 2 illustre une autre forme de réalisation de l'appareil de soins corporels 1 selon laquelle ce dernier comprend une tête de travail 3 qui n'est pas amovible et qui est pourvue en tant que moyens de traitement 4 de deux rouleaux 27 et 28 dont un au moins est entraîné par un moteur 29 piloté par l'unité de commande UC. Un tel appareil est par exemple conçu pour réaliser des passages selon une technique dite du palpé-roulé. Selon cet exemple, les moyens 10 de détection de proximité de la peau sont situés dans un espace entre les rouleaux 27 et 28 et ne mettent pas en oeuvre de guide de lumière directement au contact avec la peau P. De plus, selon cet exemple, les moyens de détection de présence 20 comprennent une source de lumière distincte de la source de lumière 11 des moyens 10 de détection de proximité. Le pilotage de l'appareil de soins corporels est assuré par l'unité de commande UC de manière sensiblement analogue à celle décrite en relation du fonctionnement de l'appareil selon la figure 1.

Bien entendu, diverses autres modifications peuvent être apportées à l'invention dans le cadre des revendications annexées.

## Revendications

1. Appareil à main de soins corporels comprenant :
- un corps (2) qui comprend une zone de préhension (Zp),
- une tête de traitement (3) qui équipe le corps (2) et qui est destinée à être appliquée contre ou à proximité de la peau et comprend des moyens (4) de traitements activés électriquement,
- des moyens (10) de détection de la proximité de la peau avec la tête de traitement,
- une unité électronique de commande (UC) qui est adaptée pour, au moins, autoriser le fonctionnement des moyens de traitement si de la peau (P) est détectée à proximité de la tête de traitement (3),
**caractérisé en ce qu'**il comprend des moyens (20) de détection de la présence d'une main sur la zone de préhension (Zp) et **en ce que** l'unité électronique de commande (UC) est adaptée pour au moins autoriser le fonctionnement des moyens de traitement (4) si de la peau (P) est détectée à proximité de la tête de traitement (3) et si conjointement une main est détectée au niveau de la zone de préhension (Zp).

2. Appareil à main de soins corporels selon la revendication 1, **caractérisé en ce que** l'unité électronique de commande (UC) est adaptée pour déclencher le fonctionnement des moyens de traitement (4) si de la peau est détectée à proximité de la tête de traitement et si conjointement une main est détectée au niveau de la zone de préhension.

3. Appareil à main de soins corporels selon la revendication 1 ou 2, **caractérisé en ce que** l'unité électronique de commande (UC) est adaptée pour autoriser ou déclencher le fonctionnement des moyens de traitement (4) si, de plus la présence de la main est détectée avant la présence de la peau.

4. Appareil à main de soins corporels selon l'une des revendications 1 à 3, **caractérisé en ce que** les moyens de détection de proximité (10) comprennent au moins une source de lumière (11) en direction d'une zone (Zd) de détection de proximité située au niveau de la tête de traitement et au moins un capteur (12) de la lumière en provenance de la zone de détection de proximité (Zd).

5. Appareil à main de soins corporels selon la revendication 4, **caractérisé en ce que** la source de lumière (11) et le capteur de lumière (12), des moyens de détection de proximité, sont situés à l'intérieur du corps (2) à distance de la tête de traitement (3) et **en ce que** l'appareil comprend au moins un guide de lumière (14, 15) interposé entre, d'une part, la source de lumière (11) et/ou le capteur (12) et, d'autre part, la zone de détection (Zd).

6. Appareil à main de soins corporels selon la revendication 5, **caractérisé en ce que** la tête de traitement (3) est amovible et **en ce que** chaque guide de lumière (14, 15) comprend une première partie solidaire du corps (2) et une deuxième partie solidaire de la tête de traitement (3).

7. Appareil selon l'une des revendications 1 à 6, **caractérisé en ce que** les moyens de détection de présence (20) comprennent au moins une source (11, 30) de lumière en direction d'une zone de détection de présence située au niveau de la zone de préhension (Zp) et au moins un capteur (22) de la lumière en provenance de la zone de détection de présence.

8. Appareil à main de soins corporels selon la revendication 7, **caractérisé en ce que** la source de lumière (11) et le capteur de lumière, des moyens détection de présence, sont situés à l'intérieur du corps à distance de la zone de préhension (Zp) et **en ce que** l'appareil comprend au moins un guide de lumière (21) interposé entre, d'une part, la source de lumière et le capteur et, d'autre part, la zone de préhension.

9. Appareil à main de soins corporels selon la revendication 5 ou 6 et la revendication 7 ou 8, **caractérisé en ce que** les moyens de détection de proximité (10) et les moyens de détection de présence (20) possèdent une source de lumière (11) en commun.

10. Appareil à main de soins corporels selon l'une quelconque des revendications précédentes **caractérisé en ce que** les moyens de traitement (4) sont adaptés pour assurer une épilation mécanique.

11. appareil à main de soins corporels selon l'une quelconque des revendications précédentes **caractérisé en ce que** les moyens de traitement (4) sont adaptés pour assurer une épilation par exposition à des radiations magnétiques, par exemple une épilation laser.

## Patentansprüche

1. Handgerät für die Körperpflege, umfassend:
- einen Körper (2) mit einem Greifbereich (Zp),
- einen Behandlungskopf (3), mit dem der Körper (2) ausgestattet ist und der auf die Haut oder in die Nähe der Haut gebracht wird und elektrisch einzuschaltende Behandlungsmittel (4) umfasst,
- Mittel (10) für die Erfassung der Nähe der Haut zum Behandlungskopf,
- eine elektrische Steuereinheit (UC), die geeignet ist, um mindestens den Betrieb der Behandlungsmittel zuzulassen, wenn Haut (P) in der Nähe des Behandlungskopfes (3) erkannt wird, **dadurch gekennzeichnet, dass** es Mittel (20) für die Erfassung des Vorhandenseins einer Hand im Greifbereich (Zp) umfasst und dass die elektrische Steuereinheit (UC) geeignet ist, um mindestens den Betrieb der Behandlungsmittel (4) zuzulassen, wenn Haut (P) in der Nähe des Behandlungskopfes (3) und zugleich eine Hand im Greifbereich (Zp) erkannt werden.

2. Handgerät für die Körperpflege nach Anspruch 1, **dadurch gekennzeichnet, dass** die elektrische Steuereinheit (UC) geeignet ist, um den Betrieb der Behandlungsmittel (4) auszulösen, wenn Haut in der Nähe des Behandlungskopfes und zugleich eine Hand im Greifbereich erkannt werden.

3. Handgerät für die Körperpflege nach Anspruch 1 oder 2, **dadurch gekennzeichnet, dass** die elektrische Steuereinheit (UC) geeignet ist, um den Betrieb der Behandlungsmittel (4) zuzulassen oder auszulösen, wenn vor der Erfassung des Vorhandenseins der Haut das Vorhandensein der Hand erkannt wird.

4. Handgerät für die Körperpflege nach einem der Ansprüche 1 bis 3, **dadurch gekennzeichnet, dass** die Mittel (10) für die Erfassung der Nähe mindestens eine Lichtquelle (11) in Richtung eines Bereichs (Zd) für die Erfassung der Nähe am Behandlungskopf und mindestens einen Sensor (12) für das Licht aus dem Bereich (Zd) für die Erfassung der Nähe umfassen.

5. Handgerät für die Körperpflege nach Anspruch 4, **dadurch gekennzeichnet, dass** sich die Lichtquelle (11) und der Lichtsensor (12), Mittel für die Erfassung der Nähe, im Inneren des Körpers (2) entfernt vom Behandlungskopf (3) befinden und dass das Gerät mindestens eine Lichtführung (14, 15) zwischen einerseits der Lichtquelle (11) und/oder dem Sensor (12) und andererseits dem Bereich (Zd) für die Erfassung umfasst.

6. Handgerät für die Körperpflege nach Anspruch 5, **dadurch gekennzeichnet, dass** der Behandlungskopf (3) abnehmbar ist und dass jede Lichtführung (14, 15) einen ersten fest mit dem Körper (2) verbundenen Teil und einen zweiten fest mit dem Behandlungskopf (3) verbundenen Teil umfasst.

7. Gerät nach einem der Ansprüche 1 bis 6, **dadurch gekennzeichnet, dass** die Mittel (20) zur Erfassung des Vorhandenseins mindestens eine Lichtquelle (11, 30) in Richtung eines Bereichs für die Erfassung des Vorhandenseins im Greifbereich (Zp) und mindestens einen Sensor (22) für das Licht aus dem Bereich für die Erfassung des Vorhandenseins umfassen.

8. Handgerät für die Körperpflege nach Anspruch 7, **dadurch gekennzeichnet, dass** sich die Lichtquelle (11) und der Lichtsensor, Mittel für die Erfassung des Vorhandenseins, im Inneren des Körpers entfernt vom Greifbereich (Zp) befinden und dass das Gerät mindestens eine Lichtführung (21) zwischen einerseits der Lichtquelle und dem Sensor und andererseits dem Greifbereich umfasst.

9. Handgerät für die Körperpflege nach Anspruch 5 oder 6 und Anspruch 7 oder 8, **dadurch gekennzeichnet, dass** die Mittel (10) zur Erfassung der Nähe und die Mittel (20) zur Erfassung des Vorhandenseins über eine gemeinsame Lichtquelle (11) verfügen.

10. Handgerät für die Körperpflege nach einem der vorhergehenden Ansprüche, **dadurch gekennzeichnet, dass** die Behandlungsmittel (4) geeignet sind, um eine mechanische Epilation zu gewährleisten.

11. Handgerät für die Körperpflege nach einem der vorhergehenden Ansprüche, **dadurch gekennzeichnet, dass** die Behandlungsmittel (4) geeignet sind, um eine Epilation durch Einwirkung magnetischer Strahlung zu gewährleisten, zum Beispiel eine Laserepilation.

## Claims

1. A handheld body care appliance, including:
- a body (2) with a gripping area (Zp),
- a treatment head (3) fitted to the body (2) to be applied to or next to the skin and with an electrically activated treatment means (4),
- a means (10) for detecting the proximity of the skin to the treatment head,
- and an electronic control unit (UC) suitable for at least authorising the operation of the treatment means if skin (P) is detected near the treatment head (3), **characterised in that** it includes a means (20) for detecting the presence of a hand on the gripping area (Zp) and **in that** the electronic control unit (UC) is suitable for at least authorising the operation of the treatment means (4) if skin (P) is detected near the treatment head (3) and if a hand is detected on the gripping area (Zp).

2. Handheld body care appliance according to claim 1, **characterised in that** the electronic control unit (UC) is suitable for activating the operation of the treatment means (4) if skin is detected near the treatment head and if a hand is detected on the gripping area.

3. Handheld body care appliance according to claim 1 or 2, **characterised in that** the electronic control unit (UC) is suitable for authorising or activating the operation of the treatment means (4) if the presence of a hand is detected before the presence of the skin.

4. Handheld body care appliance according to one of claims 1 to 3, **characterised in that** the proximity sensing means (10) include at least a light source (11) facing a proximity sensing zone (Zd) located near the treatment head and at least a light sensor (12) from the proximity sensing zone (Zd).

5. Handheld body care appliance according to claim 4, **characterised in that** the light source (11) and the light sensor (12), with proximity sensing means, are located inside the body (2) away from the treatment head (3) and **in that** the appliance includes at least a light guide (14, 15) located between, firstly, the light source (11) and/or the sensor (12) and, secondly, the sensing zone (Zd).

6. Handheld body care appliance according to claim 5, **characterised in that** the treatment head (3) is removable and **in that** each light guide (14, 15) includes firstly a body section (2) and secondly a treatment head section (3).

7. Appliance according to one of claims 1 to 6, **characterised in that** the presence sensing means (20) include at least a light source (11, 30) facing a presence sensing zone located near the gripping zone (Zp) and at least a light sensor (22) from the presence sensing zone.

8. Handheld body care appliance according to claim 7, **characterised in that** the light source (11) and the light sensor, with presence sensing means, are located inside the body away from the gripping zone (Zp) and **in that** the appliance includes at least a light guide (21) located between, firstly, the light source and the sensor and, secondly, the gripping zone.

9. Handheld body care appliance according to claim 5 or 6 and claim 7 or 8, **characterised in that** the proximity sensing means (10) and the presence sensing means (20) have a common light source (11).

10. Handheld body care appliance according to one of the previous claims, **characterised in that** the treatment means (4) are designed to ensure mechanical hair removal.

11. Handheld body care appliance according to one of the previous claims, **characterised in that** the treatment means (4) are designed to ensure hair removal by exposure to electromagnetic radiation, e.g. laser hair removal.
